# EUROPEAN PATENT SPECIFICATION

(11) **EP 3 188 684 B1**
(45) Date of publication and mention of the grant of the patent: **25.12.2019**
(21) Application number: 15771261.3
(22) Date of filing: 21.08.2015
(51) Int. Cl.: A61C 1/08, A61C 8/00

(54) **SURGICAL TEMPLATE FOR DENTAL AND/OR ORTHODONTIC IMPLANTS AND METHOD FOR DESIGNING A SURGICAL TEMPLATE**
CHIRURGISCHE SCHABLONE FÜR ZAHN- UND/ODER KIEFERORTHOPÄDISCHE IMPLANTATE UND VERFAHREN ZUM ENTWURF EINER CHIRURGISCHEN SCHABLONE
GABARIT CHIRURGICAL POUR IMPLANTS DENTAIRES ET/OU ORTHODONTIQUES, ET PROCÉDÉ DE CONCEPTION D'UN GABARIT CHIRURGICAL

(30) Priority: 02.09.2014 IT VI20140220
(43) Date of publication of application: 12.07.2017
(60) Divisional application: 18000332.9
(73) Proprietor: Maino Dott. Bortolo Giuliano, 36100 Vicenza (IT); Orthomodul Di Paoletto Emanuele, 36016 Thiene (VI) (IT)
(72) Inventor: MAINO, Bortolo Giuliano, I-36100 Vicenza (IT); PAOLETTO, Emanuele, I-36016 Thiene (VI) (IT)
(74) Representative: Maiello, Helenio Francesco
(86) International application number: PCT/IB2015/056346
(87) International publication number: WO 2016/034973

(56) References cited:
- WO-A1-2008/014097
- WO-A1-2013/092744
- US-A1- 2012 214 121
- KIM ET AL: "Surgical positioning of orthodontic mini-implants with guides fabricated on models replicated with cone-beam computed tomography", AMERICAN JOURNAL OF ORTHODONTICS AND DENTOFACIAL ORTHOPEDICS, MOSBY, ST. LOUIS, MO, US, vol. 131, no. 4, 11 April 2007 (2007-04-11), pages S82-S89, XP022029253, ISSN: 0889-5406, DOI: 10.1016/J.AJODO.2006.01.027
- PAREL S M ET AL: "Interactive imaging for implant planning, placement, and prosthesis construction", JOURNAL OF ORAL AND MAXILLOFACIAL SURGERY, SAUNDERS, PHILADELPHIA, PA, US, vol. 62, 1 September 2004 (2004-09-01), pages 41-47, XP004590902, ISSN: 0278-2391, DOI: 10.1016/J.JOMS.2004.05.207
- JANGHYUN PAEK ET AL: "Virtually fabricated guide for placement of the C-tube miniplate", AMERICAN JOURNAL OF ORTHODONTICS AND DENTOFACIAL ORTHOPEDICS, vol. 145, no. 5, 1 May 2014 (2014-05-01), pages 694-702, XP055189923, ISSN: 0889-5406, DOI: 10.1016/j.ajodo.2013.02.036
- THOMAS BERNHART ET AL: "Alternative to the median region of the palate for placement of an orthodontic implant", CLINICAL ORAL IMPLANTS RESEARCH, vol. 11, no. 6, 1 December 2000 (2000-12-01), pages 595-601, XP055190408, ISSN: 0905-7161, DOI: 10.1034/j.1600-0501.2000.011006595.x
- COUSLEY R: "Critical aspects in the use of orthodontic palatal implants", AMERICAN JOURNAL OF ORTHODONTICS AND DENTOFACIAL ORTHOPEDICS, MOSBY, ST. LOUIS, MO, US, vol. 127, no. 6, 1 June 2005 (2005-06-01), pages 723-729, XP004927495, ISSN: 0889-5406, DOI: 10.1016/J.AJODO.2004.01.027
- MARTIN ET AL: "Template Fabrication for a Midpalatal Orthodontic Implant: Technical Note", INTERNATIONAL JOURNAL OF ORAL AND MAXILLOFACIAL IMPLANTS,, vol. 17, no. 5, 1 January 2002 (2002-01-01), pages 720-722, XP009184330, ISSN: 1399-0020
- TOSUN T ET AL: "Method for the placement of palatal implants", INTERNATIONAL JOURNAL OF ORAL AND MAXILLOFACIAL IMPLANTS, QUINTESSENCE PUBL., CHICAGO, IL, US, vol. 17, no. 1, 1 January 2002 (2002-01-01), pages 95-100, XP009184328, ISSN: 0882-2786
- THOMAS FORTIN ET AL: "An Image-Guided System-Drilled Surgical Template and Trephine Guide Pin to Make Treatment of Completely Edentulous Patients Easier: A Clinical Report on Immediate Loading", CLINICAL IMPLANT DENTISTRY AND RELATED RESEARCH, vol. 6, no. 2, 1 July 2004 (2004-07-01), pages 111-119, XP055190356, ISSN: 1523-0899, DOI: 10.1111/j.1708-8208.2004.tb00033.x

## Description

### Technical Field

The present invention finds its application in dentistry and relates with a method for designing a surgical template for dental and/or orthodontic implants.

The invention also relates with a surgical template for dental and/or orthodontic implants suitable to be manufactured with said designing method.

### State of the art

In dental surgery, the computer-aided surgical implantology is constantly evolving as it allows the designing of dental implant placements based on precise measurements of the anatomy of each one patient.

Such operative methodology, while presenting on the one hand greater complexity and higher costs compared to traditional techniques, has certain advantages with regard of the matching of the implant morphology with the patient anatomy and of the precision in the positioning thereof, making the whole therapy more reliable and safe, as well as more quick.

Typically, computer-aided methods for programming implants and for manufacturing template for positioning thereof are directed to the programming of implants deisgned for prosthetic uses and to orthodontic mini-implants to be placed inside of the alveolar process.

A known technique of designing an implant site, cited in WO2014/040695, provides for the acquisition of an image or a scan of the oral cavity of the patient in order to realize the imprint and subsequently the stereolithographic model according to any one of the techniques currently available.

In this way it is possible to configure the prosthesis directly on the model, adjusting the shape and position thereof and then place it *in situ* and possibly make further adjustments based directly on the anatomy of the patient.

These starting steps have the object of providing the radiographic guide for the implant, which will require a subsequent step of scanning and capturing the images of soft tissue and bone tissue of the patient by means of a computed tomography.

In particular a computer model of the patient's mouth is obtained by a first scan, while a 3D model of the bone structure and soft tissue of the patient is obtained by a second scan.

The two models are subsequently superimposed to obtain the overall set on which it is possible to design the insertion of the implant and subsequently build the template provided with holes with guides for the positioning of the respective implants.

However, this procedure is slightly convenient and subject to different error possibilities, both during the adjusting steps and in the steps of acquisition of the images by scanning or tomography, with the result that the patient may be forced to be subjected to more sessions during which he undergoes X-ray.

Not least, even if the template thus obtained allows to place the implant according to a programmed inclination, it is not possible to predefine the maximum depth stroke of the implant, which must always be controlled by the surgeon and therefore strictly depends on the skill thereof.

In the same document cited above it is also described a further method of designing the implant which comprises a step of acquiring a 3D image of a surface which represents the mouth of the patient, a step of defining by computer a model reproducing the template designed to snugly fit the oral cavity of the patient, a step of defining the position of the holes for the insertion of the implants and subsequent modification of the computer model of the template to introduce therein the guides for the implants in correspondence of the holes.

In this way the final model of the template is get, which can be physically manufactured according to stereolithography techniques or by 3D printing.

However, even this method is not free from drawbacks, in particular due to the fact that the design of the implant, i.e. the step of defining of diameter and inclination, is carried out on computer models that do not consider the whole composition of the portion of the oral cavity, but it is only based on the computerized image of the template.

In addition, the design stop of the guide holes for the implants is very laborious and involves a series of steps and modifications of the computer model, necessary to remove the material of the template, to reinforce the same template in correspondence of the holes and to provide the presence of an adhesive substance for the fixing of the guides to the template, which are somewhat complex and susceptible to errors.

Not least, it is also necessary a further step of modifying the computer model of the template in order to ensure that the same can be removed in an easy way.

Further examples of methods for computer-aided designing of dental implants, always adapted to be inserted inside of the maxillary or mandibular bones, are also described in US5768134, EP1486900 and WO2008043056.

Janghyun Paek et Al. "Virtually fabricated guides for placement of the C-tube miniplate" (American Journal of Orthodontics and Dentofacial Orthopedics - Vol. 145) discloses a system for the virtual design and the stereolithography making-of of templates for the application of orthodontic implants which allows the accurate planning of mini-implants to be inserted in the alveolar process through a method starting from the elaboration of data obtained from a plaster model in order to obtain a virtual 3D model of the dentition.

The 3D picture of the pattern is subsequently repositioned on a 2D image of the dentition, acquired through teleradiography, so that the virtual model replace the toothing itself, so as to proceed to the design of the site of insertion of the implant.

The surgical guide is initially created on the screen and then realized using stereolithography techniques.

Also in this case, however, the technique suffers from relatively high inaccuracy because of the combination of 2D images with 3D images.

Moreover, it does not consider the fact that X-ray images acquired with the traditional cephalometric not duplicate the anatomical areas with the exact 1:1 ratio, but, by contrast, they provides images slightly enlarged.

Further, none of the techniques described above takes into account that the palatal vault can also constitute a useful zone of application of miniscrews and/or mini implants, as well as of osseointegrated implants mainly designed for orthodontic purposes and/or for anchoring prosthetic articles.

Although the palatal vault has not radicular structures that can interfere with the application of mini-implants or implants, however, bone thickness varies greatly from a patient to one other by decreasing as you move rearwardly and laterally with respect to the median palatine suture. Anatomical structures such as the lower wall or floor of the nose, the palatal nose foramen, the apex of the front teeth and the maxillary sinus may be violated by the insertion of implants, mini implants and/or miniscrews because the common radiographic inspections (X-ray and intraoral orthopantomography) used in dentistry for the insertion of the implants do not give reliable and accurate information for surgical applications in this seat.

Therefore, in the surgical designing of these devices, the technician has to select the most appropriate site both forward and rearward in order to use the bone thickness in an appropriate way to the biomechanical features of the implants and/or of the orthodontic article and, then, he/she has to vary the inclination of the inclusion depending on the availability of the bone and of the features of the orthodontic and/or prosthetic superstructure.

If then the implant devices need parallelism when applied, it could be necessary to rely mainly on the experience of the technician.

Therefore, in order to apply devices such as implants, mini-implants and/or mini screws or the like, to be inserted in bone structures of the palatine vault, with reliability and safety, it is necessary to develop more efficient working methods for the design and the production of surgical guides for computer aided oral surgery.

Kim et al. "Surgical positioning of orthodontic mini-implants fabricated with guides on models replicated with cone-beam computed tomography" (American Journal of Orthodontics and Dentofacial Orthopedics - Vol. 131) discloses the use of cone beam computed tomography (CBCT) for the acquisition of the slice of the back jaw and for the placement of the implants.

### Scope of the invention

The object of the present invention is to overcome the above mentioned drawbacks, by providing a method for designing surgical templates for orthodontic and/or dental implants that is characterized by high accuracy and efficiency.

A particular object is to provide a method for designing surgical template for orthodontic and/or dental templates which is fast, precise and which takes account of the complete conformation of the maxillary bone of the patient, so as to ensure also that the implants are placed parallel to each other and at the correct depth.

A particular object is to provide a method for designing a surgical template that ensures true reproduction of the measurements detected to the computer during radiology and in the mouth models, in particular at the time of the realization of the guides.

Still another object is to provide a surgical template for orthodontic and/or dental implants that may be used for the application of one or more implants directly to the palate.

A particular object is to provide a surgical template for orthodontic and/or dental implants in which the implants guides facilitate the precise positioning of the implants, in the regulation both of the angular position that of the depth of insertion.

A further object is to provide a surgical template which ensures maximum visibility of the implant for the surgeon during the whole application step, so as to allow a visual check of the exact designed depth of application.

Still another object is to provide a surgical template which is has low invasiveness and is easily removable after the insertion of the implant, whatever the shape of the implant and/or mini-implant.

These objects, and others which will appear more clearly hereinafter, are achieved by a method for computer-aided designing of a surgical template for dental implants that, according to claim 1, comprises the steps of acquiring a first 2D or 3D image of the oral cavity of the patient, processing said first image for defining of one or more second 2D images reproducing corresponding views or sections of the oral cavity with respect to at least one sagittal plane, realizing a digital model of the upper dental arch and of the palate of the patient and identifying one or more suitable sites for insertion of a respective implant, selecting one or more slices of said digital model in correspondence with one or more sagittal planes passing through respective said one or more insertion sites identified in advance, overlapping said one or more slices with corresponding second 2D images for the definition of a third 2D image, providing a digital library of implants, selecting the implants from said library, virtual positioning of said implants in said third image, adjusting the angle and/or the depth of insertion of said virtual implants.

Thanks to this combination of steps, the operator will have one or more pairs of 2D images respectively of the anatomy of the oral cavity and of the model of the dental arch perfectly adapted to overlap with each other, with the possibility to know with high precision the conformation of the anatomy of the oral cavity to determine the most suitable areas in which to apply the implants, depending on the availability of bone.

The method according to the invention allows to realize guides with high accuracy and sized in function of the detected anatomy of the patient.

In this way it is possible to select implants with the most appropriate dimensions both in length and diameter and at the same time to select the areas of the palate with the greater availability of bone for placing implants having the minimum predetermined optimum length in order to ensure the firm positioning of implants and prosthetic devices or devices for dental correction to be connected to such implants.

Also, it is possible to adjust in a simple, immediate and precise way both the angle of the installations and their depth of insertion to replicate these measurements in the guides which the template will be provided with so as to ensure also the parallelism between the implants. According to a first embodiment of the method, the first image is a 3D image acquired by computed tomography, preferably of the cone beam type (CBCT).

The superimposition of a digital model reproducing the part of the oral cavity which the implants have to be applied to a digital image of the same oral cavity in which the bone structure is visible, for example acquired by computed tomography (CT), also of the cone beam type (CBCT), allows the surgeon to define the most suitable sites for placing the implants also taking into account the amount of available bone.

According to an alternative mode of carrying out the method, said first image may be a 2D image acquired by radiography, said processing step comprising a reduction with predetermined reduction coefficient to bring it to a 1:1 ratio with respect to the effective anatomy of the patient.

In this case, the superposition of a digital model reproducing the part of the oral cavity to which the implants have to be applied to radiographic 2D images of the same oral cavity, such as a latero-lateral and/or anterior-posterior teleradiography, will allow the operator to define even in this case the most suitable sites for the insertion of the implants taking into account the amount of available bone, the thickness of the soft tissues of the palate and the length of the transgingival portion of the implant, the inclination of the same implant and exposing the patient to a lower dose of radiation.

According to a further aspect of the invention there is provided a surgical template for dental implants, in particular for palatal implants, adapted to be manufactured preferably but not exclusively by a method according to claim 1.

The template, according to claim 7, comprises a palatal portion reproducing the shape of the palate of a patient and at least one substantially cylindrical guide formed in said palatal portion and having an axial passage with an inlet section and an outlet section for the ' insertion of a corresponding orthodontic implant in the palate of the patient with predetermined angle and depth.

The guide has end-stroke means adapted to define an axial abutment for the implant when it is inserted in said passage for adjusting said insertion deepness.

This will make it possible to guarantee the correct positioning of the implant or mini-implant inside of the bone, thus simplifying the whole process of application, whose success will not closely linked to the greater or lesser skill of the surgeon.

Conveniently, said axial passage may comprise an inlet cylindrical length of the implant and a substantially frusto-conical outlet length defining said end-stroke means and adapted to snugly fit the head of the implant to lock it at a predetermined height.

In this way, the insertion deepness of the implant may be defined directly during the design step on the basis of the selected implant and in particular in function of the shape and dimensions thereof.

Advantageously, said palatal portion or the only guide may be optically transparent or translucent.

Furthermore, said at least one guide may have a perimeter slot that may enable the vision within said passage and to define a reference for the insertion of the implant at the correct deepness.

Thanks to this combination of features, the surgeon may verify during the entire insertion step of the implant the advancement and position thereof, avoiding the implant is inserted with too great deepness or with not sufficient deepness in particular in those cases wherein the implant could be blocked in its advancing due to increased bone strength or any other obstacles that may give the impression that they have reached the end-stroke.

Advantageously, said at least one guide may be connected to said palatal portion by means of connection areas with reduced strength and/or pre-cut lines adapted to make easy its removal from the palate.

Advantageous forms of the invention are obtained according to the dependent claims.

### Brief description of the drawings

Further features and advantages of the invention will become more apparent in light of the detailed description of some preferred but not exclusive embodiments of a surgical template of the invention and of two embodiments of a method for designing a template, illustrated by way of non-limiting example with the aid of the accompanying drawing, wherein:
**FIG. 1** is a perspective view of a template of the invention in a first embodiment;
**FIG. 2** is a front view of the template of Fig. 1;
**FIG. 3** is a front view of a first correction device anchored by implants adapted to be applied by means of the template of Fig. 1;
**FIGG. 4a, b, c** shows three imagines relative to corresponding designing steps of the template of the invention in a second embodiment;
**FIG. 5** is a front view of a second corrective device anchored by means of implants adapted to be applied with the template of Fig. 4;
**FIG. 6** is a first partial perspective view of the template of Fig. 1 applied to the palate and during the positioning step of a implant;
**FIG. 7** is a second partial perspective view of the template of Fig. 1 applied to the palate and during the positioning step of a implant;
**FIG. 8** is a further front view of the template of Fig. 1 during the positioning of a pair of implants;
**FIG. 9** is an exploded perspective view of an implant adapted to be used with a template of the invention;
**FIGs. 10** to **12** show three different embodiments of a device for inserting the implant;
**FIGs. 13** to **21** show the steps for carrying out a method of the invention in a first preferred embodiment;
**FIGs. 22** to **28** show the steps for carrying out a method of the invention in a second preferred embodiment.

### Best modes of carrying out the invention

With reference to the accompanying figures, there is illustrated a surgical template for positioning orthodontic implants in the oral cavity of a patient.

Although in the figures there is shown a template in association with orthodontic implants, in particular for applications in the palate, the same template may also be used for positioning prosthetic implants or other removable or non-removable types of implants.

In its most general embodiment, a surgical template for orthodontic implants, generally indicated by **1,** comprises a palatal central portion **2** with an upper surface **3** which reproduces in negative the shape of the palate of a patient to be positioned in contact therewith in a precise manner.

In addition, the template **1** has a support peripheral portion **4** which preferably reproduce at least partially the shape of the dental arch of the patient to be lean thereon, and in particular on the surface of occlusion of the posterior teeth, so as to ensure the stability of the template **1** during use.

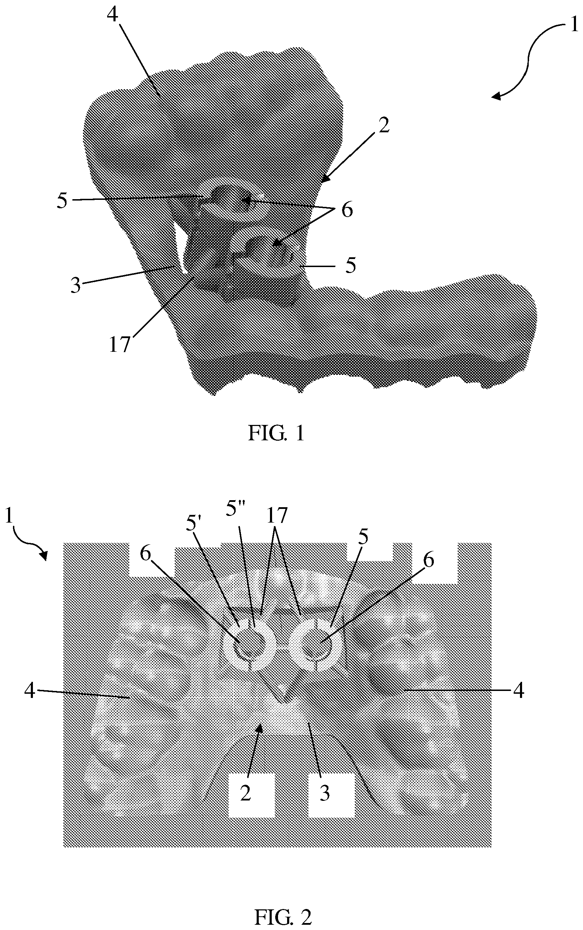

As visible from **Fig. 1****,** two substantially cylindrical guides **5** are made in the palatal portion **2** each having a respective axial passage **6** with an inlet section **7** and an outlet section **8** for the insertion of a corresponding dental implant **9** designed to be set in the palate of the patient with predetermined angle and deepness.

The two guides **5** have substantially identical dimensions with respective passages **6** having central extension axes **X** as much as possible parallel to each other to ensure uniformity in the positioning of the implants **9,** necessary for the correct performance of the therapy.

From **Fig. 2** it could be observed that the two guides **5** are laterally and mutually staggered in side-by-side position with a predetermined distance **d** between the respective central extension axes **X.**

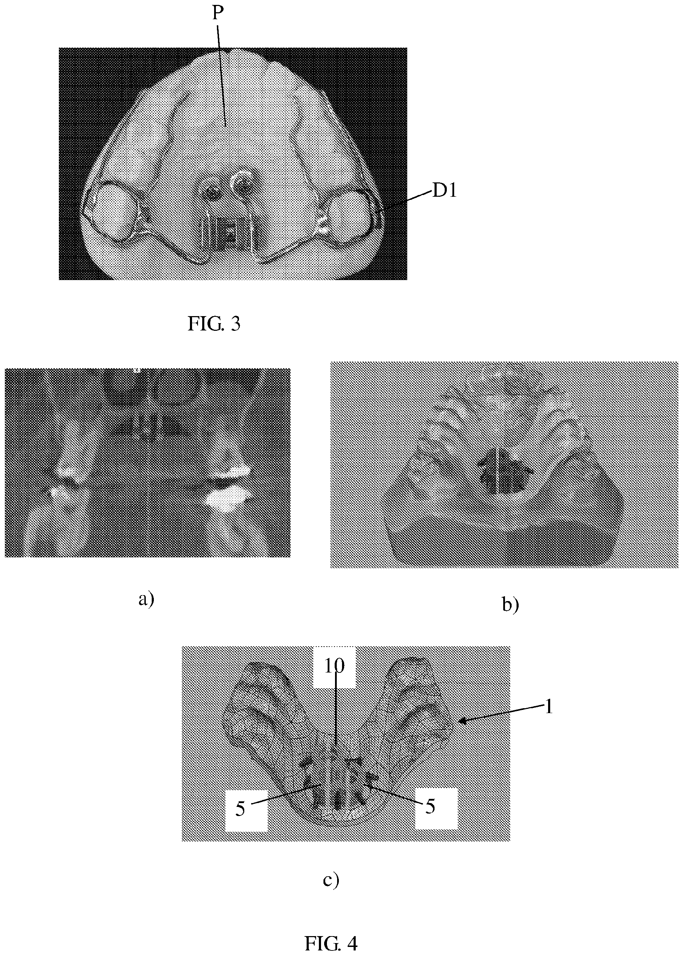

**Fig. 3** shows a particular embodiment of a corrective device **D1** fixed to the palate **P** by a pair of implants **9** positioned by means of the template **1** described above.

**Fig. 4** shows a second embodiment of the template **1** which differs from that described above in particular for the fact that it further comprises, in addition to said pair of guides **5,** a third guide **10** disposed in the forward position with respect to one of the side guides **5.**

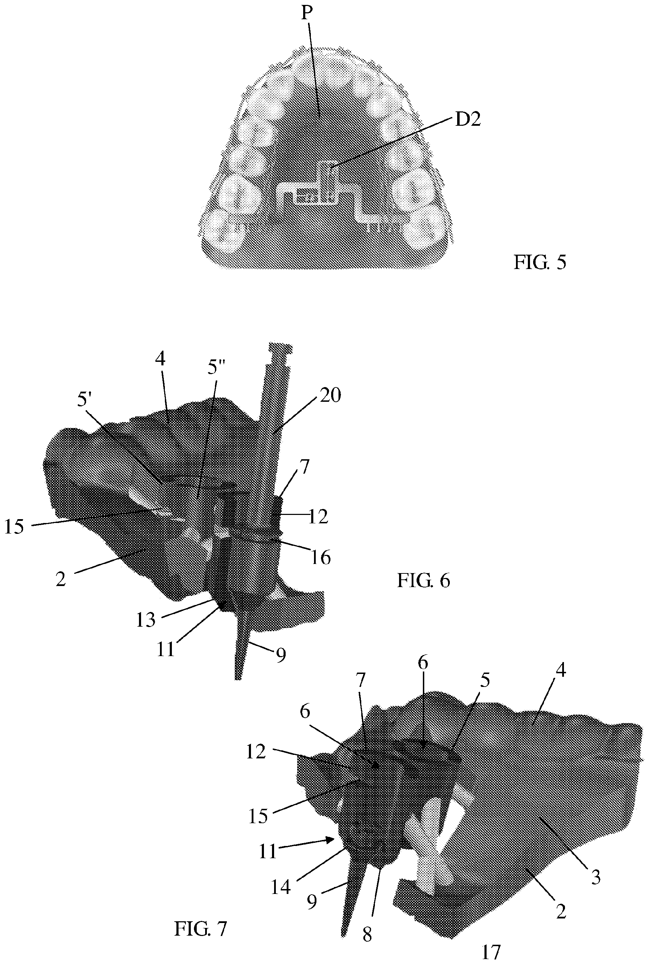

This embodiment would be particularly useful for positioning a corrective device **D2** as shown in **Fig. 5****.**

Regardless of the number of guides provided for the specific template, advantageously each of these guides **5, 10** comprises respective end-stroke means **11** adapted to define an axial abutment for the implant **9** at the time of its insertion in the respective passage **6,** so as to allow the adjustment of the insertion deepness within the bone.

In a preferred but not exclusive manner, the axial passage **6** of each guide **5** may have a substantially cylindrical inlet length **12** wherein the implant **9** could be inserted and an outlet length **13** which defines a constriction.

According to the preferred embodiment shown in **Figs. 6** and **7****,** referred to the template **1** according to the first embodiment, the axial passage **6** of each guide **5** comprises a substantially cylindrical inlet length **12** in which the implant **9** could be inserted and an outlet length **13** with a substantially frusto-conical shape whose lateral wall define the end-stroke means **11.**

In particular, the frusto-conical outlet length **13** will be suitably dimensioned during the design step to snugly fit a conical head of the specific selected implant, e.g. the transmucosal collar **14** of a miniscrew for orthodontic anchorage **9,** to lock it at a predetermined height also selected during design step as function of the amount of available bone, as will become more clear in the following description of the method.

If the used implant has a portion of transmucosal collar with cylindrical shape, the outlet length **13** may be also cylindrical with an axial abutment for the collar.

Regardless of the specific configuration of the end-stroke means, the guides **5** will have respective perimeter windows **15** adapted to allow viewing inside the corresponding passages **6** and to define a reference for the insertion of the implant **9** at the correct deepness.

More precisely, through the window **15,** the operator will have the opportunity to verify that the implant **9** has actually reached the end-stroke **11** and has not been blocked by any obstacles, such as areas of bone with greater resistance.

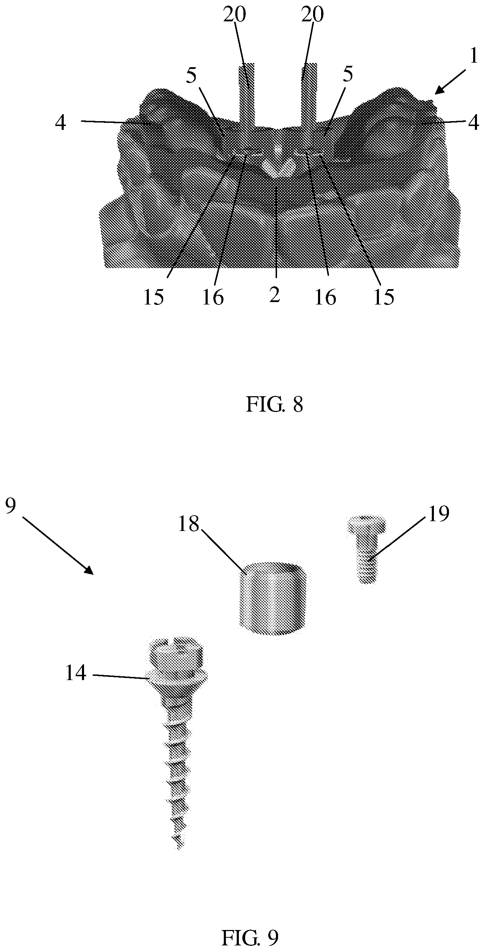

To this end, as shown more clearly from **Fig. 8****,** the implant **9** or the tool for its screwing may be provided with a reference element **16** suitably sized to be placed at the window **15** when the implant **9** is in the correct position.

In particular, it is possible to use implants or mini-implants and tools of the type commonly available on the market and the axial position of the window **15** may be determined in the design step of the template **1** as a function of detected measures.

In order to further increase the visibility inside the guides **5,** even in the absence of the windows **15,** the palatal portion **2,** or only the guides **5** may be made of an optically transparent or translucent material, such as transparent resins for 3D printing. Advantageously, the whole template **1** may be in an optically transparent or translucent material.

To make the removal of the template **1** easier when the implants **9** have been applied, the guides **5** will be connected to the palatal portion **2** by means of areas with structural weakening **17** adapted to define connection lines with reduced strength.

For example, according to a first configuration, not shown, the weakening areas will be pre-cut lines, or, as in the embodiments of the figures, each of the guides **5** will be formed by a pair of semi-cylindrical portions **5', 5"** opposite to each other to define the corresponding passage **6** and radially offset from each other so as to be partially or completely separated along the whole axial extension.

In this way between the two semi-cylindrical portions **5', 5"** a further axial slot will be defined to facilitate even more the control of the moving forward of the implants inside of the passages **6.**

The two semi-cylindrical portions **5', 5"** are connected to the palatal portion **2** by respective and distinct weakness areas **17** defined by a plurality of bridges or beams made of resin or other polymeric material that will ensure sufficient stability to the connection between the guides and the palatal portion but which at the same time may easily be cut to allow removal of the template **1** and of the guides **5.**

In particular, the staggered half-cylinders shape for the guides **5** will allow the simple removal from the implant **9** upon the breaking of the bridges **17.**

**Fig. 9** shows a particular embodiment, exemplifying and not limiting of the invention, of a implant **9** adapted to be inserted in the palate by means of a template **1** of the invention.

The illustrated implant **9** is a miniscrew for orthodontic anchorage of the Spider screw regular Plus type, whose orthodontic head **14** may be associated with an abutment element **18** fixable to the orthodontic head **14** by means of a fixing screw **19** to allow the stable connection of the correction devices to the implants.

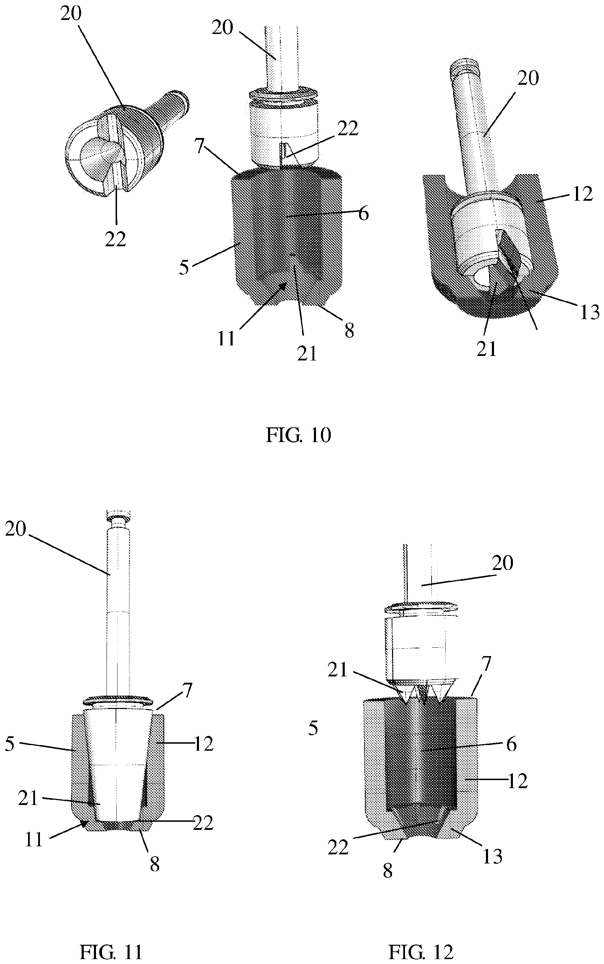

**Figs. 10** to **12** show alternative embodiments of the guides **5** and of the end stroke means **11** which may be either associated with a template **1** according to the present invention, or advantageously implemented also in further templates, in particular in templates not designed according to the method of the present invention or templates not provided of the above weakening areas and/or the above windows.

In general in these further embodiments the end stroke means may be associated with both the outlet length **13** of the guides **5** and the tool **20** for inserting the implant **9.**

In particular, the end stroke means **11** will be of the male and female type with a male element associated to one between the outlet length **13** of the guide **5** and the head of the tool **20** and a female element associated with the other between the outlet length **13** of the guide **5** and the head of the tool **20** so as to be engaged by the male element only when the implant **9** is in the right designed position.

In the embodiment of **Fig. 10** the guides **5** have a passage **6** totally cylindrical and the end stroke means **11** comprise a male element **21** defined by an abutment tooth placed in correspondence of the outlet section **8.** The outlet length **13** may be either cylindrical or tapered as in the figure.

The male element **21** is adapted to interact with a corresponding female abutment element **22** associated with the head of the tool **20** so that the tooth **21** engages the abutment element **22** upon a movement of roto-translation of the tool **20** and to the achievement of the design predetermined deepness.

In this way, when the implant will be inserted with the correct deepness, the above abutment element **22** will impact on the abutment tooth **21** preventing further rotation.

**Fig. 11** shows a further embodiment in which the outlet length **13** of the guide **5** has a conical narrowing **21** with a radial abutment surface for the conical head **22** of the tool **20.**

In **Fig. 12** the head of the tool **20** will be provided with shaped radial projections **21** realized on the peripheral wall of its head to be inserted into corresponding recesses defining the female elements **22,** countershaped with respect of the radial projections.

Also in this case the outlet length **13** may indifferently be either cylindrical or tapered as in figures.

However, such embodiments are solely exemplifying and not restrictive and in particular further configurations may be provided in which the male and female elements may be differently shaped and arranged.

For example, an additional configuration, not shown, may have a cylindrical passage **6** of the guide **5** provided with a perimetric protrusion which narrows the lumen of the passage at a predetermined height so as to create an abutment for the head of the tool screwing the implant.

As matter of fact, since the tool is wider than the implant itself, it will be only the latter to pass, while the abutment will prevent the head of the tool to further penetrate, so that the same will be locked.

Moreover, the abutment screw may also be performed by inserting in the cylindrical guides **5** one or more metal rings reducing the lumen of the guide **5** and always acting in abutment against the screwing tool.

The rings may be different in thickness, or height and operate as thickness gauges to lock the descent of the screwing tool.

In a further configuration the passage 6 is cylindrical and the head of the tool 20 is provided with a flat abutment which counter-act in correspondence of the inlet front section **7** of the guide.

The template described above in the different embodiments may be designed and manufactured according to any of the known techniques, without particular limitations.

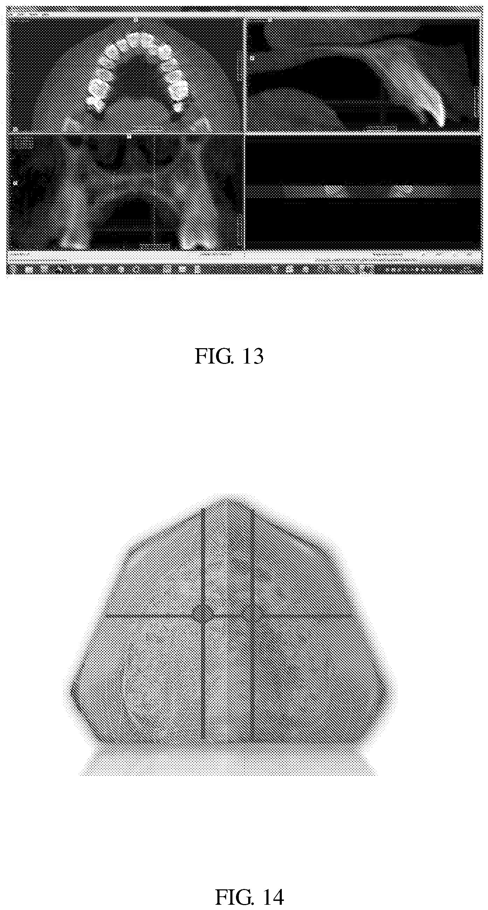

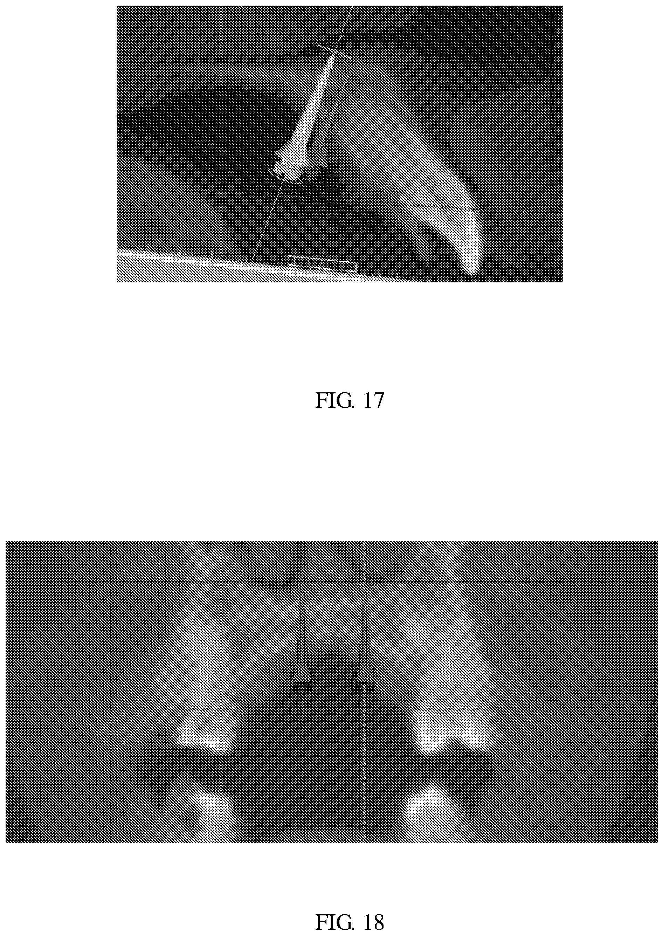

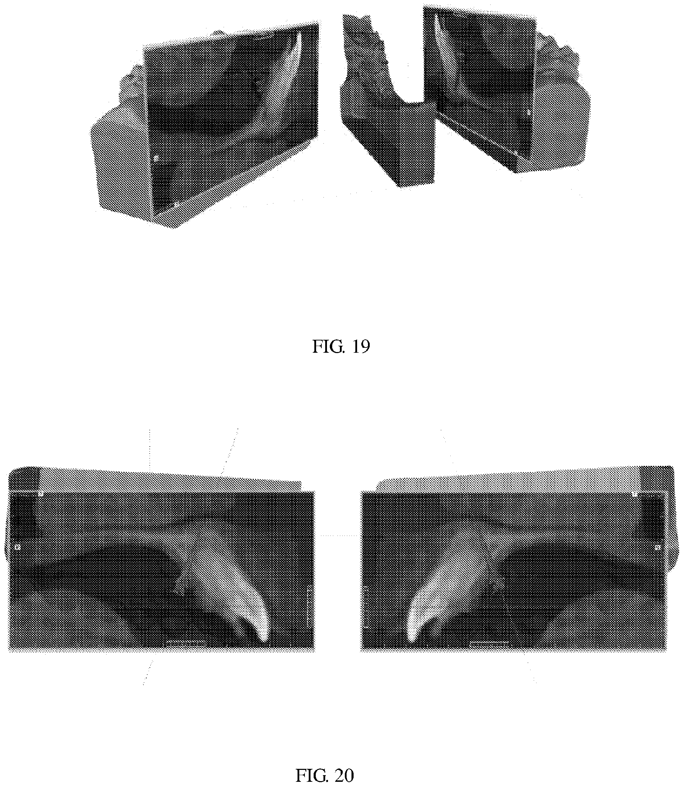

**Figs. 13** to **21** show a particular preferred, but not exclusive, embodiment of a method for computer-aided designing of surgical template for dental implants.

This method may advantageously be used both for designing and manufacturing the template according to the present invention that for templates of known type, or other types of templates.

The method will be particularly suitable for the realization of templates for palatal anchorages as the palate does not present uniform thickness but has a conformation highly variable from person to person.

Therefore in this type of applications it needs to get the most accurate measurements, taking into account the real bone conformation of the palate.

The method provides a step a) of acquiring a first 2D or 3D image in the oral cavity of the patient (**Fig. 13**).

The object of this step is to obtain an image as more reliable as possible of the structure of the jawbone and will preferably be carryied out by means of a CAT or even more preferably by a technique of computed tomography cone beam (CBCT- *Cone Beam Computed Tomography*). This will make it possible to elaborate the first image to generate one or more second 2D digital images, preferably according to the DICOM standard, in order to identify the anatomical structure of the palate and identify the most suitable areas of the palate for implant placement (step b).

In particular, the processing step b) includes a step b') of identifying one or more areas of the palate on the first image, preferably a pair of areas, suitable to receive a respective implant, and a step b") of selecting respective second 2D images at parasagittal planes passing through the selected areas.

The second images will reproduce corresponding views or sections of the oral cavity with respect of a parasagittal plane.

At the same time a digital model of the dental arch and of the palate of the patient may be realized (step c), according to any known technique, which may be obtained either directly by a intraoral scanner or making first an imprinting of the arch and then scan it to get the digital model.

One or more suitable sites for insertion of a respective implant may be identified on the digital model thus obtained to subsequently select (step d) one or more slices of the digital model in correspondence with one or more parasagittal planes passing through respective insertion sites previously identified.

In this way further digital 2D images will be obtained, which may be converted into a STL file, on which one or more ideal points for the positioning of the implants may be identified.

The points will be preferably detected in areas meeting the biomechanical needs, for example in an area between the distal surface of the canines and the mesial surface of the premolars, also considering that usually the distance between the two points is close to 10mm (**Fig. 14**) and also considering the design configuration of the device to be associated to the implant.

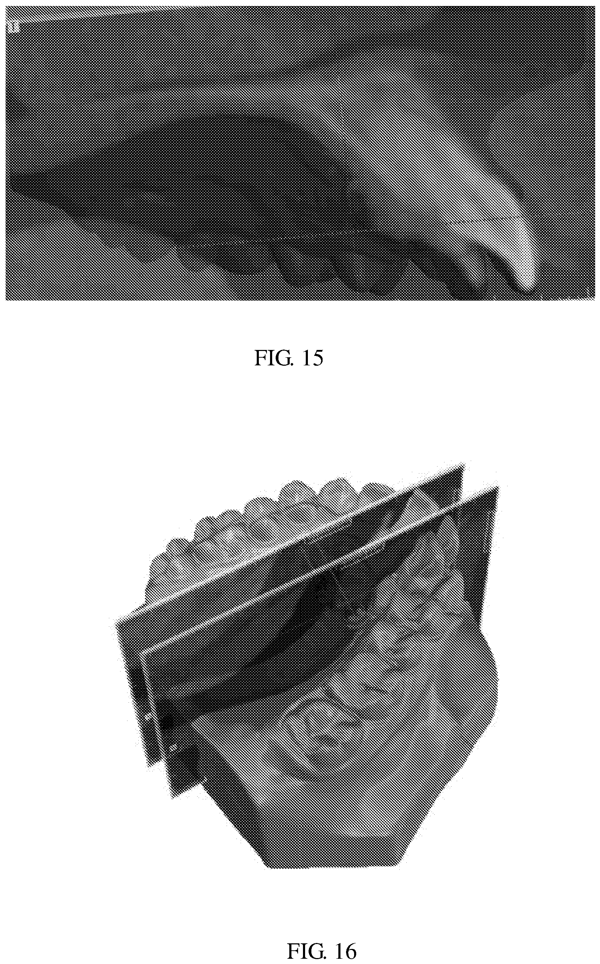

In a subsequent step e) the slices are overlapped with the corresponding second digital image, i.e. to the DICOM file (**Fig. 15** and **Fig. 16**) for defining a third complex 2D digital image.

In this way it will be possible to identify the most suitable spatial position for the micro-screws on the basis of the deepness and thickness of the palate.

In particular, it could identify both the transverse plane that parasagittal planes corresponding to the sites of insertion of implants.

In this way the virtual position of the implant or miniscrews inside the third digital complex image at respective slice CBCT images (**Figg.16-20**) will be possible.

The implant or the implant having the greater length compatible with the amount of available bone at previously identified positioning sites may be selected by adjusting the position of the virtual plants within a digital library appropriately prepared (step f and step g).

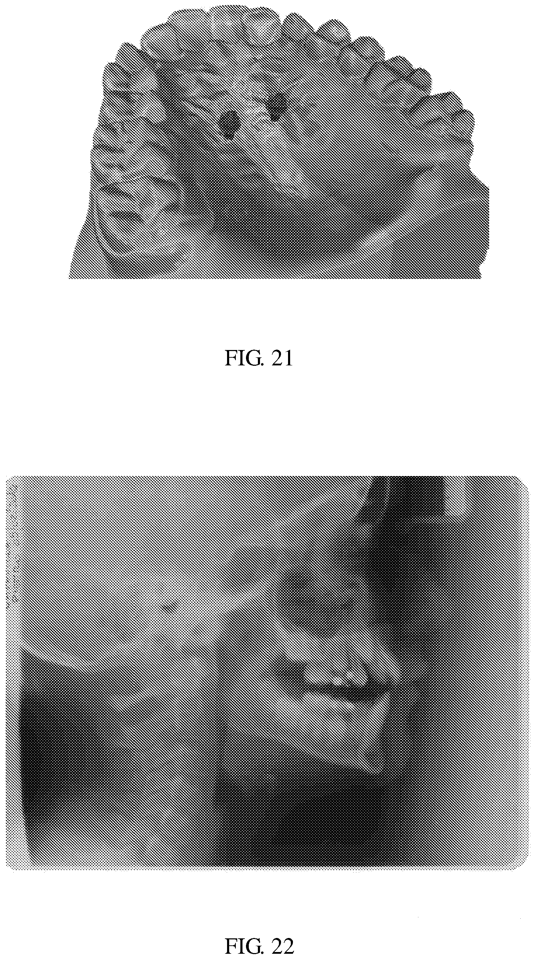

At this point the three-dimensional check of the application of mini-screws on 3D model occurs in order to assess the adequacy of the length of the screws and or mini-implants and that they are parallel (**Fig. 21**), also ensuring the availability of bone at the points of intersection of the transverse plane with the parasagittal planes.

Subsequently, you can proceed to the design of the template, for example of the type illustrated in Fig. 1, taking into account that the same must rest on the surface of occlusion of the posterior teeth and starting from the design of the positioning guides of the implants.

The use of STL images of the miniscrews allows to faithfully reproduce the measures that have to be reported with the utmost precision in the design of the guides, in particular at the length **13** designed to house the transmucosal collar of the implant and the portion designed for the tool insertion.

These measures will be transferred to the inner part of the cylindrical guides **5** which replicate the previously predetermined angle of insertion and which will be configured to prevent that the implant can be introduced with excessive or lower deepness than the needs.

Finally, any resin bridges or equivalent weakness areas may be virtually determined. According to an alternative configuration, not illustrated, the cylindrical guides **5** may be provided with connection means to the template **1** of the bayonet, screw/nut or the like type to allow the coupling by screwing or movement of roto-translation. In this way the cylinder of the guide **5,** which can also be in metal and can be prefabricated in standard size, is screwed into the individual template and will thread up to the deepness designed in the previous processing of the 2D and 3D images.

Then, the metal cylinder acts as a guide and as a stop for the insertion of the implant and/or of the miniscrew.

The virtual model of the template so designed may be used for the realization of the real template with 3D printing techniques, stereolithography techniques or according to any technique suitable for the purpose.

The insertion of the implants and application of the fixed or removable prostheses or devices of correction may be performed according to any methods of known type and are not described in this text because it does not fall within the scope of the present invention.

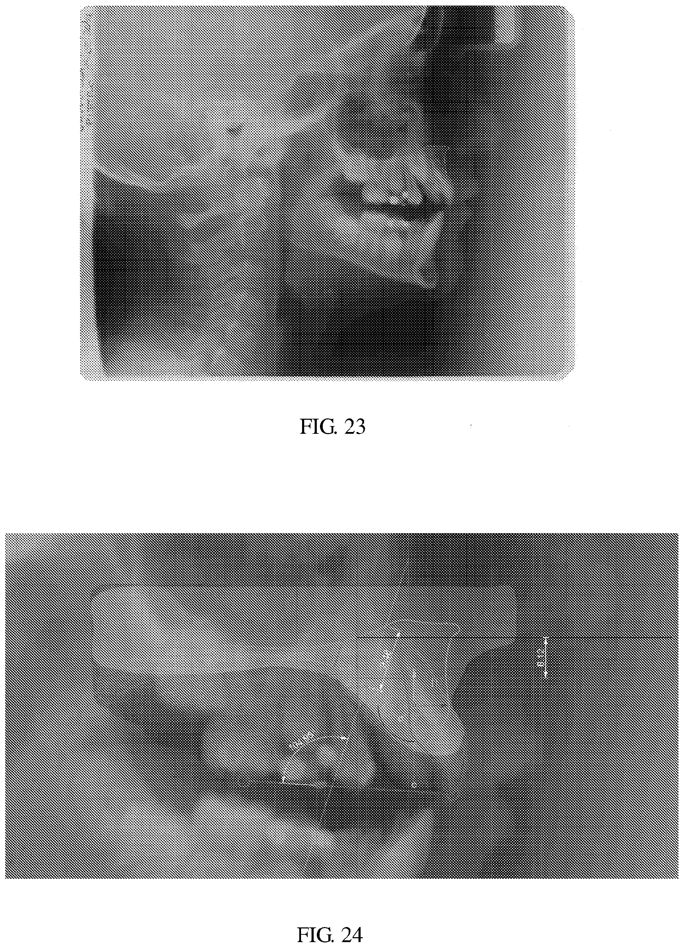

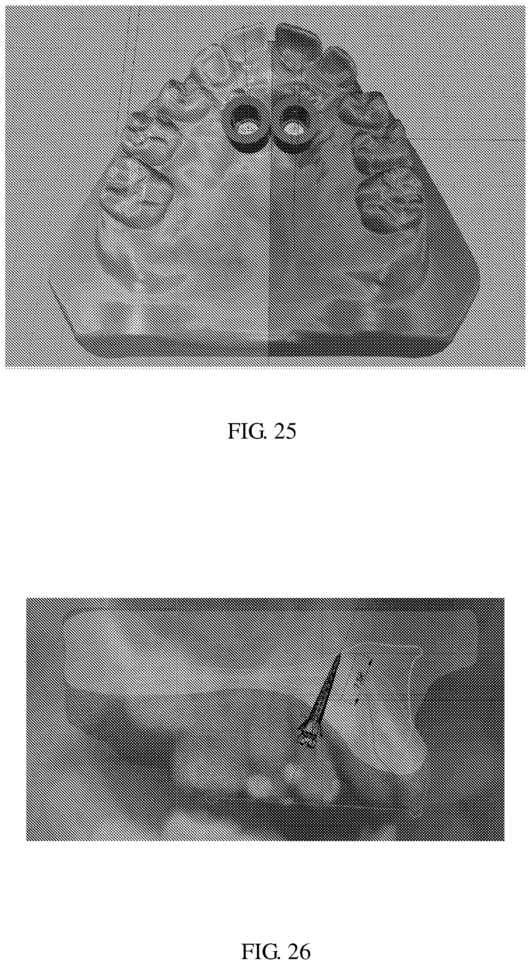

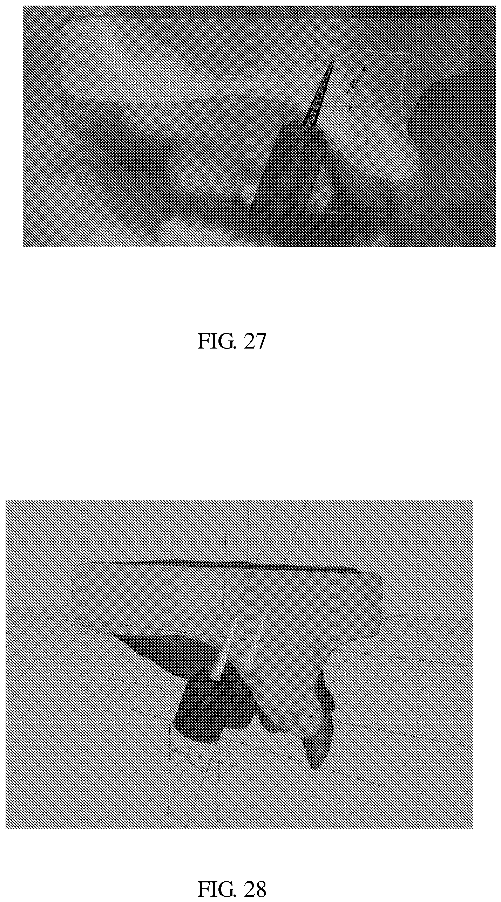

**Figs. 22** to **28** show a variant of the method described above, which differs from that described above first of all for the fact that the step a) of acquisition of the first image is a two-dimensional x-ray of the oral cavity, such as a latero-lateral and/or anterior-posterior teleradiography.

The first 2D image so acquired will be subsequently processed (step b) to be reduced with a predetermined reduction coefficient and brought to a 1:1 ratio with respect to the real anatomy of the patient.

After making the digital model of the patient's mouth the median sagittal plane of the model will be selected for generating a single slice to be overlapped on the first 2D image processed and get the third image on which the implants will be designed and in particular to define the deepness and/or angle thereof in the manner described above to then proceed to the design of the guides, in a manner substantially identical to that described above.

However, in this case if it is decided to apply two implants, they will be arranged in positions substantially symmetrical with respect to the median sagittal plane at a distance selected according to anatomical features of the palate.

From above it appears evident that the template and its design method according to the invention reach the intended objects.

The template and its design method according to the invention are susceptible of numerous modifications and variations, all falling within the inventive concept expressed in the accompanying claims. All the details may be replaced with other technically equivalent elements, and the materials may be different according to requirements, without departing from the scope of the present invention.

Although the template and the method have been described with particular reference to the attached figures, reference numbers used in the description and in the claims are used to improve the intelligence of the invention and do not constitute any limitation the claimed scope.

## Claims

1. A computer-aided method for designing a surgical template for dental and/or orthodontic implants, **characterized by** comprising the following steps:
a) acquiring a first 2D or 3D image of the oral cavity and of the overlying anatomical structures of a patient;
b) processing said first image for defining one or more second 2D images showing corresponding views or sections of the oral cavity with respect to a sagittal plane;
c) realizing a digital model of the upper dental arch and of the palate of the patient and identifying one or more suitable sites for inserting a corresponding implant;
d) selecting one or more slices of said digital model at one or more sagittal or parasagittal planes passing through respective of said one or more insertion sites previously identified;
e) overlapping said one or more slices with corresponding second 2D images for generating a third 2D image;
f) providing a digital library of implants;
g) selecting the implants from said library;
h) virtual positioning of said implants into said third image;
i) adjusting the inclination and/or the deepness of insertion of said virtual implants;
said computer-aided method being **characterised in that** said processing step b) comprises a step b') for identifying one or more areas, preferably a pair of areas, of the palate on said first image, which areas being suitable to receive a respective implant and a step b") for selecting respective second 2D images at sagittal or parasagittal planes passing thorough said selected areas.

2. Method as claimed in claim 1, **characterized in that** said first image is a 3D image captured by a computerized tomography, preferably of the cone beam type.

3. Method as claimed in claim 1, **characterized in that** said first image is a 2D image captured by a radiography, said processing step b) comprising a reduction with predetermined reduction to bring it to a 1:1 ratio with respect of the real anatomy of the patient.

4. Method as claimed in any preceding claim, **characterized in that** the implants are selected from said library in function of the size and amount of the available bone at said previously identified positioning areas.

5. Method as claimed in any preceding claim, **characterized by** comprising, downstream of said step i), a step j) for designing the guides for positioning the implants in function of said size, inclination and/or deepness of said implants, a final step 1) being also provided for designing the definitive model.

## Patentansprüche

1. Computergestütztes Verfahren zum Entwerfen einer Operationsschablone für zahnärztliche und / oder kieferorthopädische Implantate, **gekennzeichnet durch** die folgenden Schritte:
a) Erfassen eines ersten 2D- oder 3D-Bildes der Mundhöhle und der darüber liegenden anatomischen Strukturen eines Patienten;
b) Verarbeiten des ersten Bildes zum Definieren eines oder mehrerer zweiter 2D-Bilder, die entsprechende Ansichten oder Schnitte der Mundhöhle in Bezug auf eine Sagittalebene zeigen;
c) Realisieren eines digitalen Modells des oberen Zahnbogens und des Gaumens des Patienten und Identifizieren einer oder mehrerer geeigneter Stellen zum Einsetzen eines entsprechenden Implantats;
d) Auswählen einer oder mehrerer Schichten des digitalen Modells in einer oder mehreren sagittalen oder parasagittalen Ebenen, die jeweils **durch** eine oder mehrere der zuvor identifizierten Insertionsstellen verlaufen;
e) Überlappen der einen oder mehreren Schichten mit entsprechenden zweiten 2D-Bildern zum Erzeugen eines dritten 2D-Bildes;
f) Bereitstellung einer digitalen Bibliothek von Implantaten;
g) Auswählen der Implantate aus der Bibliothek;
h) virtuelles Positionieren der Implantate in dem dritten Bild;
i) Einstellen der Neigung und / oder der Tiefe des Einsetzens der virtuellen Implantate;
wobei das computerunterstützte Verfahren **dadurch** gekennzeichnet ist, dass der Verarbeitungsschritt b) einen Schritt b') zum Identifizieren eines oder mehrerer Bereiche, vorzugsweise eines Paares von Bereichen, des Gaumens auf dem ersten Bild umfasst, wobei diese Bereiche geeignet sind, ein jeweiliges Implantat aufzunehmen und einen Schritt b") zum Auswählen jeweiliger zweiter 2D-Bilder in sagittalen oder parasagittalen Ebenen, die durch die ausgewählten Bereiche verlaufen.

2. Verfahren nach Anspruch 1, **dadurch gekennzeichnet, dass** das erste Bild ein 3D-Bild ist, das mit einer Computertomographie, vorzugsweise vom Typ eines Kegelstrahls, aufgenommen wurde.

3. Verfahren nach Anspruch 1, **dadurch gekennzeichnet, dass** das erste Bild ein durch Radiographie aufgenommenes 2D-Bild ist, wobei der Verarbeitungsschritt b) eine Verkleinerung mit vorbestimmter Verkleinerung umfasst, um es in Bezug auf die reale Anatomie auf ein Verhältnis von 1:1 zu bringen des Patienten.

4. Verfahren nach einem der vorhergehenden Ansprüche, **dadurch gekennzeichnet, dass** die Implantate in Abhängigkeit von der Größe und Menge des verfügbaren Knochens an den zuvor identifizierten Positionierungsbereichen aus der Bibliothek ausgewählt werden.

5. Verfahren nach einem der vorhergehenden Ansprüche, **dadurch gekennzeichnet, dass** nach dem Schritt i) ein Schritt j) zum Entwerfen der Führungen zum Positionieren der Implantate in Abhängigkeit von der Größe, Neigung und / oder Tiefe der Implantate ein Endprodukt umfasst wird Schritt 1) auch zum Entwerfen des endgültigen Modells vorgesehen sein.

## Revendications

1. Procédé assisté par ordinateur pour la conception d'un modèle chirurgical d'implants dentaires et/ou orthodontiques, **caractérisé en ce qu'**il comprend les étapes suivantes:
a) acquérir une première image 2D ou 3D de la cavité buccale et des structures anatomiques sus-jacentes d'un patient;
b) traiter ladite première image pour définir une ou plusieurs secondes images 2D montrant des vues ou des coupes correspondantes de la cavité buccale par rapport à un plan sagittal;
c) réaliser un modèle numérique de l'arcade dentaire supérieure et du palais du patient et identifier un ou plusieurs sites appropriés pour l'insertion d'un implant correspondant;
d) sélectionner une ou plusieurs tranches dudit modèle numérique sur un ou plusieurs plans sagittaux ou parasagittaux passant à travers l'un ou l'autre desdits sites d'insertion précédemment identifiés;
e) superposer lesdites une ou plusieurs tranches aux deuxièmes images 2D correspondantes pour générer une troisième image 2D;
f) fournir une bibliothèque numérique d'implants;
g) sélectionner les implants à partir de ladite bibliothèque;
h) positionnement virtuel desdits implants dans ladite troisième image;
i) ajuster l'inclinaison et/ou la profondeur d'insertion desdits implants virtuels;
ledit procédé assisté par ordinateur étant **caractérisé en ce que** ladite étape de traitement b) comprend une étape b') pour identifier une ou plusieurs zones, de préférence une paire de zones du palais sur ladite première image, lesquelles zones sont appropriées pour recevoir un implant respectif et une étape b") pour sélectionner des secondes images 2D respectives sur des plans sagittaux ou parasagittaux passant à travers lesdites zones sélectionnées.

2. Procédé selon la revendication 1, **caractérisé en ce que** ladite première image est une image 3D capturée par une tomographie informatisée, de préférence du type à faisceau conique.

3. Procédé selon la revendication 1, **caractérisé en ce que** ladite première image est une image 2D capturée par une radiographie, ladite étape de traitement b) comprenant une réduction avec une réduction prédéterminée pour l'amener à un rapport 1:1 par rapport à l'anatomie réelle du patient.

4. Procédé selon l'une quelconque des revendications précédentes, **caractérisé en ce que** les implants sont choisis parmi ladite bibliothèque en fonction de la taille et de la quantité de l'os disponible au niveau desdites zones de positionnement identifiées précédemment.

5. Procédé selon l'une quelconque des revendications précédentes, **caractérisé en ce qu'**il comprend, en aval de l'étape i), une étape j) de conception des guides pour positionner les implants en fonction de la taille, de l'inclinaison et/ou de la profondeur desdits implants, une l'étape 1) étant également prévue pour concevoir le modèle définitif.
